# EUROPEAN PATENT APPLICATION

(11) **EP 1 284 468 A2**
(43) Date of publication of application: **19.02.2003**
(21) Application number: 02352010.9
(22) Date of filing: 22.03.2002
(51) Int. Cl.: G06F 19/00

(54) **Medical record management system**

(30) Priority: 17.08.2001 JP 2001006211 U
(71) Applicant: Makino Ophtalmic Instrument Co., Ltd., Tokyo 113-0033 (JP)
(72) Inventor: Makino, Toshimi, Sumida-ku, Tokyo (JP)
(74) Representative: Morelle, Guy Georges Alain

(57) **Abstract**

It is an object of the present invention to obtain a simple and low-cost system that can instantaneously transmit details of a medical record prepared by a doctor to a plurality of information terminals located at remote sites. A medical record management system 10 comprises wireless imaging apparatus 11 (wireless CCD camera) that imports a medical record 1 or 1A that has been handwritten or entered onto a personal computer by a doctor, as image data, by photographing same; medical record management and control means 13 that has a reception means 12 that uses wireless communication to receive an image photographed by this imaging apparatus 11 as information data; data recording means 14 that is connected to this medical record management and control means 13 for storing the information data; and a display means 18 or output means 19 that receives the information data sent via wired or wireless information transmission means 16 from the medical record management and control means 13 and either displays or prints out that information data.

## Description

This invention relates to a medical record management system in which a medical record prepared by a doctor during a consultation or the like can be recorded as electronic data such as image or text data, and then this electronic data can be sent to an accounting or testing department or a pharmacy located in a remote location.

Medical records handwritten by doctors during examination of patients are generally taken by a nurse or hospital employee into the accounting department for undergoing accounting procedure. Further, patients themselves take referrals to a testing department or prescriptions to a pharmacy so that they take the test or receive the medication.

The aforementioned medical records are stored in order in a cabinet after diagnosis and treatment has ended.

However, in the conventional medical record management system described above, time is required to take the medical records or prescriptions to other departments and a problem arises that a patient has to wait a very long time. Also, from a hospital perspective, space is required for a waiting room for patients. This is inefficient and requires additional costs.

Also, even if medical records are stored in numerical order, over a period of years the order is often lost and a great deal of effort is required to find a record. Sometimes they may even become lost.

In recent years with the major progress in information processing technology, large hospitals have constructed information transmission paths, such as LANs, within the hospital. A doctor uses an information processing terminal to create electronic medical records by directly entering particulars of diagnosis and treatment as data. These are then processed in order, by an accounting department for example, and the time for which a patient must wait is reduced. In addition, the hospital gains an efficient flow of patients, and waiting space and car parking space can be effectively utilised.

However, when a LAN such as described above is constructed, doctors must enter data in an information processing terminal and it is difficult for some elderly doctors to do this. Furthermore, handwritten medical records may also be required and therefore the burden on the doctors is increased.

Furthermore, for smaller hospitals or clinics, it is too costly to construct a LAN and the conventional methods have been the only alternative.

In light of such circumstances, an object of the present invention is to provide a simple and low-cost system that can instantaneously transmit the details indicated on a medical record prepared by a doctor to a plurality of remote information processing terminals.

A further object of the present invention is to provide a medical record management system that uses a simple and low-cost method to enable information handwritten by a doctor in a medical record or recorded by input into a personal computer to be placed onto a data base.

To fulfil these objects, the medical record management system according to the present invention (invention of claim 1) comprises: wireless imaging apparatus that imports an image of a medical record created by a doctor by photographing same;
medical record management and control means that has receiving means for receiving images photographed by said imaging apparatus as information data via wireless communication; data recording means that is connected to said medical record management and control means for recording said information data; and an information processing terminal that receives the information data sent from said medical record management and control means via wired or wireless information transmission means, and that has display means for displaying this information data or output means for printing out this information data.

The medical record management system of the present invention (invention of claim 2), according to claim 1, is characterised in that the aforementioned medical record in a medical record management system is either text information handwritten by a doctor or display information entered into a personal computer by a doctor and displayed on a display part.

The medical record management system of the present invention (invention of claim 3), according to claim 1 or claim 2, is charactirised in that the medical record management and control comprises operation control means for controlling the wireless imaging apparatus by remote control operation.

The medical record management system of the present invention (invention of claim 4), according to claim 1, claim 2, or claim 3, is characterised in that the medical record management and control means further comprises display means for displaying information data transmitted from the wireless imaging apparatus.

The medical record management system of the present invention (invention of claim 5), according to any one of claims 1 through 4, is characterised in that the medical record management and control means comprises data conversion means for converting image data obtained by the wireless imaging apparatus into character information data, and is configured to record character information data obtained by this data conversion means into the data recording means.

The medical record management system of the present invention (invention of claim 6), according to any one of claims 1 through 5, is characterised in that the wireless imaging apparatus is a wireless CCD camera.

The present invention enables a medical record handwritten or entered into a personal computer by a doctor at the time of examination or treatment to be placed in a data base without undue effort, by using an easy and low-cost system. Furthermore, that information data can immediately be retrieved by an accounting or testing department or pharmacy in remote locations. Having the processing in every department done in advance enables a more efficient medical care system.

Also, cables for connecting display apparatus and storage apparatus are not required because wireless imaging apparatus is used. Furthermore, there are few restriction relating to the site of use and so construction of the entire system takes little effort, is low-cost, and results in user-friendly medical record management.

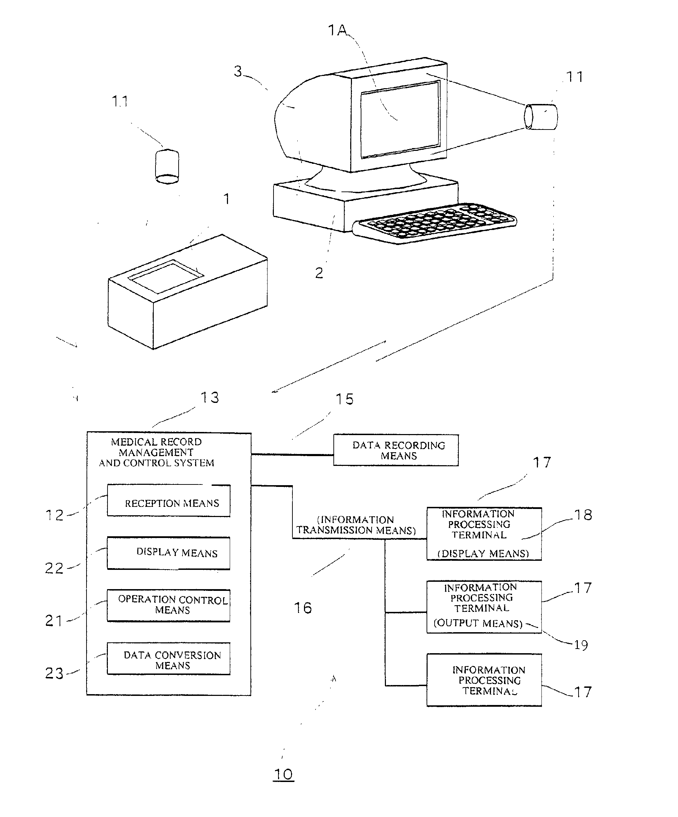

Figure 1 is a schematic view of an aspect of the embodiment of a medical record management system according to the present invention and provides an overview of the system.

Fig. 1 shows an aspect of the embodiment of the medical record management system according to the present invention. In the figure, a medical record management system 10 comprises: a wireless imaging apparatus 11, such as a wireless CCD camera, that takes images of a medical record 1 or 1A created by a doctor by photographing same; and medical record management and control means 13 that has reception means 12 for receiving images photographed by this imaging apparatus 11 as information data via wireless communication.

As shown in Fig. 1, the medical record can be either a medical record 1 in which text information is handwritten by a doctor, or a medical record 1A in which information is entered into a personal computer 2 and displayed on a display part 2a as display information.

Also, the above medical record management and control means 13 comprises operation control means 21 for controlling the wireless imaging apparatus 11 by remote operation. It is preferable that the above reception means 12 also be able to act as a sending means.

The above medical record management and control means 13 further comprises display means 22 for displaying information data transmitted from the wireless imaging apparatus 11. This enables the person in charge of system operation to be immediately informed of the management and control status.

The above medical record management and control means 13 can also comprise a data conversion means 23 for converting image data obtained by wireless imaging apparatus 11 into character information data (text information). This enables character information data in the form of text information obtained by data conversion means 23 to be recorded in data recording means 23. Thus, there are advantages in that information is easy to read and less storage capacity is required than would be the case for image data.

Data recording means 14 is also connected to the above medical record management and control means 13. It is configured to allow recording of information data transmitted from this medical record management and control means 13 via wired or wireless information transmission means 15. It is preferable, but not essential, that this data recording means 14 be installed in the same place as medical record management and control means 13. It can also be installed in a separate location. Information transmission means 15 placed in a remote location can be configured to allow appropriate sending and receiving of data by cable, public communication lines, or wireless telecommunication.

The medical record management and control means 13 can also be provided with means for searching and retrieving stored data from the above data recording means 14.

Furthermore, a multiplicity of information processing terminals 17 that receive the above information data sent via information transmission means 16 by wired or wireless telecommunication are connected to the above medical record management and control means 14. These information processing terminals 17 are installed in places such as the accounting department of a hospital, a testing department, and a pharmacy. They are set up to receive information data by any appropriate means, for example via a wired connection using cables, via public communication lines, or by wireless telecommunication. These information processing terminals 17 are fitted with display means 18, such as a monitor, for displaying this information data or with output means 19 that can print out the information.

Use of wireless apparatus (wireless CCD camera) as the imaging apparatus 11 in a medical record management system 10 of the above configuration enables information written in the medical record 1 or 1A, which is handwritten or by input into a personal computer by a doctor during diagnosis or treatment, to be placed onto a data base without undue effort on a user-friendly and low-coast system.

Particular benefits of the system are: the fact that wireless communication means can be used to transfer data from any place since the installation sites for wireless imaging apparatus 11 can be freely selected; the ability to adjust the range for taking images; and the ease of use. In addition, accounting departments, testing departments, or pharmacies in locations away from the site of the information data can immediately retrieve data from information processing terminals 17. Advance processing in each department enables the waiting time of patients to be reduced and greater efficiency in the overall medical care system to be achieved.

Also, cables connecting the system to display apparatus 22 or recording apparatus 14 are not required because the present invention uses wireless imaging apparatus 11. Furthermore, there are few restrictions regarding the sites of use and so little effort is required in construction of the overall system. This means that a simple, low-cost system that enables user-friendly medical record management is achieved.

The present invention is, however, not limited to the configuration of the aspect of the embodiment described above. The shape and structure of each part can be modified or changed as appropriate.

For example, in the aspect of the embodiment described above, detailed explanation of each part has been omitted. However, it goes without saying that the medical record control means 13 is equipped with a transceiver part that implements wireless communication with the wireless CCD camera 11.

Also, in the above aspect of the embodiment, the information processing terminals 17 to which information data from the medical record 1 or 1A is sent are located for example, in accounting departments, testing departments, or pharmacies. However, the locations of information processing terminals 17 are not restricted to these areas, with various other examples of use envisaged.

In the medical record management system according to the present invention as described above, medical record information handwritten or by input onto a personal computer by a doctor during diagnosis and treatment is placed on a data base without undue effort through the use of a simple, low-cost system. This information data can also be immediately retrieved by an accounting department, testing department, or pharmacy located in a remote location. Advance processing in each department enables a more efficient medical care system to be achieved.

Also, cables connecting the system to display apparatus or storage apparatus are not required because of the use of wireless imaging apparatus. Furthermore, because there are few restrictions relating to the sites in which the system can be used, construction of the overall system requires little effort. A simple, low-cost system is therefore obtained enabling user-friendly medical record management.

## Claims

1. A medical record management system, comprising:
wireless imaging apparatus that imports an image of a medical record created by a doctor by photographing same;
medical record management and control means that has receiving means for receiving images photographed by said imaging apparatus as information data via wireless communication;
data recording means that is connected to said medical record management and control means for recording said information data; and
an information processing terminal that receives said information data sent from said medical record management and control means via wired or wireless information transmission means, and that has display means for displaying this information data or output means for printing out this information data.

2. The medical record management system according to claim 1, wherein said medical record is either text information handwritten by a doctor or display information entered into a personal computer by a doctor and displayed on a display part.

3. The medical record management system according to either claim 1 or claim 2, wherein said medical record management and control means comprises operation control means for controlling said wireless imaging apparatus by remote control operation.

4. The medical record management system according to either claim 1, claim 2, or claim 3, wherein said medical record management and control means further comprises display means for displaying information data transmitted from said wireless imaging apparatus.

5. The medical record management system according to any one of claims 1 through 4, wherein said medical record management and control means comprises data conversion means for converting image data obtained by said wireless imaging apparatus into character information data, and is configured to record character information data obtained by this data conversion means into said data recording means.

6. The medical record management system according to any one of claims 1 through 5, wherein said wireless imaging apparatus is a wireless CCD camera.
